# EUROPEAN PATENT APPLICATION

(11) **EP 2 045 323 A1**
(43) Date of publication of application: **08.04.2009**
(21) Application number: 07450177.6
(22) Date of filing: 05.10.2007
(51) Int. Cl.: C12N 15/09, C12N 15/86, C07K 14/11, C12N 7/04

(54) **Linear expression constructs for production of influenza virus particles**

(71) Applicant: AVIR GREEN HILLS BIOTECHNOLOGY RESEARCH DEVELOPMENT TRADE AG, 1180 Vienna (AT); Medizinische Universität Wien, 1090 Wien (AT)
(72) Inventor: Wolschek, Markus, 1090 Wien (AT); Egerov, Andrej, 1180 Wien (AT); Bergmann, Michael, 3400 Klosterneuburg (AT); Muster, Thomas, 1180 Vienna (AT); Kittel, Christian, 1020 Vienna (AT)
(74) Representative: Loidl, Manuela Bettina

(57) **Abstract**

The present invention provides a linear expression construct free of any conventional amplification and/or selection sequences comprising an RNA polymerase I (poll) promoter and a poll termination signal, inserted between a RNA polymerase II (poIII) promoter and a polyadenylation signal useful for the expression of segments of viral RNA, preferably influenza viruses.
The inventive construct is useful for efficient and fast production of viral particles, especially for producing vaccine formulations for the treatment of epidemic and/or pandemic diseases.

SEQUENCE LISTING

<110>  AVIR Green Hills Biotechnology Research Development Trade
       AG
       Medical University Vienna

<120>  Novel constructs for expression of influenza virus

<130>  PCRF/EP

<160>  6

<170>  PatentIn version 3.3

<210>  1
<211>  42
<212>  DNA
<213>  Influenza A virus

<400>  1
cgaagttggg ggggagcaaa agcaggggat aattctatta ac                          42


<210>  2
<211>  44
<212>  DNA
<213>  Influenza A virus

<400>  2
gccgccgggt tattagtaga aacaagggtg tttttaatta atgc                        44


<210>  3
<211>  32
<212>  DNA
<213>  Influenza A virus

<400>  3
cctgcttttg ctccccccca acttcggagg tc                                      32


<210>  4
<211>  34
<212>  DNA
<213>  artifical sequence

<400>  4
ggggtatcag ggttattgtc tcatgagcgg atac                                   34


<210>  5
<211>  36
<212>  DNA
<213>  Influenza A virus

<400>  5
ccttgtttct actaataacc cggcggccca aaatgc                                 36


<210>  6
<211>  32
<212>  DNA
<213>  artificial sequence

<220>
<223>  plasmid sequence

<400>  6

ccccttggcc gattcattaa tgcagctggt tc 32

## Description

The present field of the invention relates to a novel linear expression construct for expressing segments of viral RNA, preferably influenza viruses, free of any amplification and/or selection sequences and comprising an RNA polymerase I (poll) promoter and a poll termination signal, inserted between a RNA polymerase II (poIII) promoter and a polyadenylation signal The invention also covers the use of this expression construct for the production of virus particles.

### Background:

Negative-strand RNA viruses are a group of animal viruses that comprise several important human pathogens, including influenza, measles, mumps, rabies, respiratory syncytial, Ebola and hantaviruses.

The genomes of these RNA viruses can be unimolecular or segmented, single stranded of (-) polarity. Two essential requirements are shared between these viruses: the genomic RNAs must be efficiently copied into viral RNA, a form which can be used for incorporation into progeny virus particles and transcribed into mRNA which is translated into viral proteins. Eukaryotic host cells typically do not contain a machinery for replicating RNA templates or for translating polypeptides from a negative stranded RNA template. Therefore negative strand RNA viruses encode and carry an RNA-dependent RNA polymerase to catalyze synthesis of new genomic RNA for assembly into progeny and mRNAs for translation into viral proteins.

Genomic viral RNA must be packaged into viral particles in order for the virus to be transmitted. The process by which progeny viral particles are assembled and the protein/protein interactions occur during assembly are similar within the RNA viruses. The formation of virus particles ensures the efficient transmission of the RNA genome from one host cell to another within a single host or among different host organisms.

Virus families containing enveloped single-stranded RNA of the negative-sense genome are classified into groups having non-segmented genomes (Paramyxoviridae, Rhabdoviridae, Filoviridae and Borna Disease Virus, Togaviridae) or those having segmented genomes (Orthomyxoviridae, Bunyaviridae and Arenaviridae). The Orthomyxoviridae family includes the viruses of influenza, types A, B and C viruses, as well as Thogoto and Dhori viruses and infectious salmon anemia virus.

The influenza virions consist of an internal ribonucleoprotein core (a helical nucleocapsid) containing the single-stranded RNA genome, and an outer lipoprotein envelope lined inside by a matrix protein (M1). The segmented genome of influenza A virus consists of eight molecules of linear, negative polarity, single-stranded RNAs which encodes eleven (some influenza A strains ten) polypeptides, including: the RNA-dependent RNA polymerase proteins (PB2, PB1 and PA) and nucleoprotein (NP) which form the nucleocapsid; the matrix membrane proteins (M1, M2); two surface glycoproteins which project from the lipid containing envelope: hemagglutinin (HA) and neuraminidase (NA); the nonstructural protein (NS1) and nuclear export protein (NEP). Most influenza A strains also encode an elevnth protein (PB1-F2) believed to have proapoptotic properties.

Transcription and replication of the genome takes place in the nucleus and assembly occurs via budding on the plasma membrane. The viruses can reassort genes during mixed infections. Influenza virus adsorbs via HA to sialyloligosaccharides in cell membrane glycoproteins and glycolipids. Following endocytosis of the virion, a conformational change in the HA molecule occurs within the cellular endosome which facilitates membrane fusion, thus triggering uncoating. The nucleocapsid migrates to the nucleus where viral mRNA is transcribed. Viral mRNA is transcribed by a unique mechanism in which viral endonuclease cleaves the capped 5'- terminus from cellular heterologous mRNAs which then serve as primers for transcription of viral RNA templates by the viral transcriptase. Transcripts terminate at sites 15 to 22 bases from the ends of their templates, where oligo(U) sequences act as signals for the addition of poly(A) tracts. Of the eight viral RNA molecules so produced, six are monocistronic messages that are translated directly into the proteins representing HA, NA, NP and the viral polymerase proteins, PB2, PB1 and PA. The other two transcripts undergo splicing, each yielding two mRNAs which are translated in different reading frames to produce M1, M2, NS1 and NEP. In other words, the eight viral RNA segments code for eleven proteins: nine structural and 2 nonstructural (NS1 and the recently identified PB1-F2) proteins.

The generation of modern vaccines for influenza viruses especially for highly pathogenic avian influenza viruses relies on the use of reverse genetics which allows the production of influenza viruses from DNA. The first development of a reverse genetic system for construction of negative-strand RNA influenza viruses involved the transfection of a single viral gene mixed with in-vitro reconstituted ribonucleoprotein (RNP) complexes and subsequent infection with an influenza helper virus. RNP complexes were made by incubating synthetic RNA transcripts with purified NP and polymerase proteins (PB1, PB2 and PA) from influenza viruses, the helper virus was used as an intracellular source of viral proteins and of the other vRNAs (Luytjes et al., 1989, Cell, 59, 1107-1113). Neumann et al. (1994, Virology, 202, 477-479) achieved RNP formation of viral model RNAs in influenza-infected cells after expression of RNA from a murine RNA polymerase I promoter-responsive plasmid.

Pleschka et al. (1996, J. Virol., 4188-4192) described a method wherein RNP complexes were reconstituted from plasmid-based expression vectors. Expression of a viral RNA-like transcript was achieved from a plasmid containing a truncated human polymerase I (poll) promoter and a ribozyme sequence that generated a 3'end by autocatalytic cleavage. The poll-driven plasmid was cotransfected into human 293 cells with poIII-responsive plasmids that express the viral PB1, PB2, PA and NP proteins. Yet, transfection efficiency was very low, approx. 10 transfectant virus particles per transfection. Additionally, this plasmid-based strategy was dependent on the aid of a helper virus.

In WO 01/04333 segmented negative-strand RNA viruses were constructed using a set of 12 expression plasmids for expressing genomic vRNA segments and RNP proteins. The vectors described in WO 01/04333 were based on well known pUC19 or pUC18 plasmids. According to the description this system requires a set of 8 plasmids expressing all 8 segments of influenza virus together with an additional set of 4 plasmids expressing nucleoprotein and subunits of RNA-dependent RNA polymerase (PB1, PB2, PA and NP).

WO 00/60050 covers a set of at least two vectors comprising a promoter operably linked to an influenza virus segment cDNA (PA, PB1, PB2, HA, NP, NA, M) linked to a transcription termination sequence and at least two vectors comprising a promoter operably linked to an influenza virus segment DNA (PA, PB1, PB2, NP). The use of a large number of different vectors was tried to overcome by using plasmids with eight RNA polymerase I transcription cassettes for viral RNA synthesis combined on one plasmid.

WO 01/83794 discloses circular expression plasmids comprising an RNA polymerase I (poll) promoter and a poll termination signal, inserted between a RNA polymerase II (poIII) promoter and a polyadenylation signal. The term vector according to this application is described as a plasmid which generally is a self-contained molecule of double-stranded DNA that can accept additional foreign DNA and which can be readily introduced into a suitable host cell.

Epidemics and pandemics caused by viral diseases are still claiming human lives and are impacting global economy. Influenza is responsible for millions of lost work days and visits to the doctor, hundreds of thousands of hospitalizations worldwide (Couch 1993, Ann. NY. Acad. Sci 685;803,), tens of thousands of excess deaths (Collins & Lehmann 1953 Public Health Monographs 213:1; Glezen 1982 Am.J.Public Health 77:712) and billions of Euros in terms of health-care costs (Williams et al. 1988, Ann. Intern. Med. 108:616). When healthy adults get immunized, currently available vaccines prevent clinical disease in 70-90% of cases. This level is reduced to 30-70% in those over the age of 65 and drops still further in those over 65 living in nursing homes (Strategic Perspective 2001: The Antiviral Market. Datamonitor. p. 59). The virus's frequent antigenic changes further contribute to a large death toll because not even annual vaccination can guarantee protection. Hence, the U.S. death toll rose from 16,363 people in 1976/77 to four times as many deaths in 1998/99 (Wall Street Journal, Flu-related deaths in US despite vaccine researches. January 7, 2003).

Especially in case of the outbreak of pandemic viral diseases, it can be of utmost importance to provide vaccinations or treatments immediately after outbreak of the disease.

In view of the urgent need for providing efficient protection and treatment of viral diseases there is a still high demand for the development of economic, fast and efficient expression systems for virus production which can overcome the disadvantages and difficulties of the present expression systems.

### Brief description of the invention

The inventors have surprisingly shown that the use of a linear expression construct free of any conventionally plasmid-based bacterial amplification and/or selection sequences comprising a viral gene cloned into a cassette of an RNA polymerase I (poll) promoter and a poll termination signal, inserted between a RNA polymerase II (poIII) promoter and a polyadenylation signal provides a highly economic and efficient tool for fast rescue of viral particles. In contrast to the plasmids used by known technologies, no cloning steps in bacterial cells are needed. Therefore, the time needed for transfection and expression of viral particles can be highly reduced, preferably from at least several weeks to few days.

For example, the linear expression construct according to the invention can be used for developing influenza viruses either of wildtype, mutant or reassortant strains. This provides a tool for fast generation of any virus vaccine needed in case of occurrence of influenza epidemics or pandemics.

In a preferred embodiment, reassortant viruses can be provided, wherein each viral segment can be selected from a specific virus strain, for example H1N1, H3N2, even H5N1 or any seasonal strain that is identified to be most relevant in causing influenza

If needed, the linear expression construct can be circularized using short linker sequences. Also methods can be provided wherein the linear expression constructs are used for the production of viral particles, or, alternatively, wherein some of the viral gene segments of a complete virus can be expressed via a circularized expression construct and at least one of the gene segments is expressed via a linearized expression construct according to the present invention.

### Figures

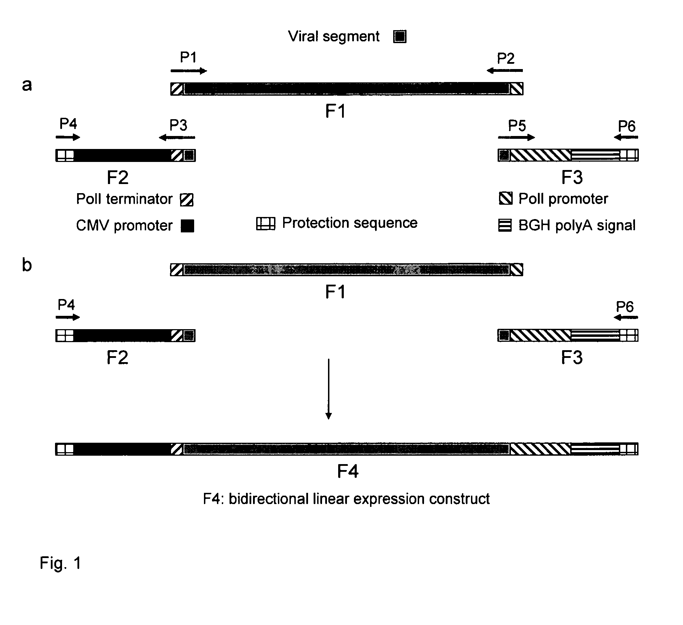

Figure 1 shows a schematic diagram of the generation of linear bidirectional expression constructs. a) Fragments F1, F2 and F3 are generated separately by PCR amplification. b) Fragment F4 is generated by overlapping PCR using the oligonucleotides P4 and P6.

### Detailed description of the invention

As already discussed in the introduction the currently used reverse genetics rescue system of segmented RNA viruses still requires the transfection of a high number of different plasmids and/or still suffers from the need for subcloning of viral genes and subsequent amplification in bacterial cells to develop a sufficient amount of plasmids. Plasmid DNA has to be sequenced, purified from bacteria for each individual clone and can only then be further used for transfection of animal cells. This method is time consuming, costly and difficult to automate.

The present invention now provides a novel, linear expression cassette free of any conventionally plasmid-based bacterial amplification and/or selection sequences comprising a viral gene cloned into a cassette of an RNA polymerase I (poll) promoter and a poll termination signal, inserted between an RNA polymerase II (poIII) promoter and a polyadenylation signal which can be used for expressing virus particles.

The inventors surprisingly showed that such inventive linear expression construct can be efficiently used for transfection of cells and expression of complete viral particles although it was often described in the art that transfection of animal cells with linear fragments can lead to fast decomposition of these fragments due to cellular exonuclease degradation (van der Aa et al. 2005, J Gene Med. 7:208-17) or result in increased apoptosis of the transfected cells (Yao et al. 2001, J Biol Chem. 276:2905-13)

The linear expression constructs do not contain any selection or amplification sequences that are needed for amplification of plasmids in bacterial cells. Neither ori (origin of replication)-sequences nor antibiotics resistance genes or any other selection markers are contained.

According to a specific embodiment of the invention the linear expression construct can comprise additional protection sequences at the N- and/or C-terminus of the construct. For example, these protection sequences can be peptide nucleic acid sequences as described in WO 00/56914. These PNAs are nucleic acid analogs in which the entire deoxyribose-phosphate backbone has been exchanged with a chemically completely different, but structurally homologous, polyamide (peptide) backbone containing 2-aminoethyl glycine units. PNA "clamps" have also been shown to increase stability, wherein two identical PNA sequences are joined by a flexible hairpin linker containing three 8-amino-3,6-dioxaoctanoic acid units. When a PNA is mixed with a complementary homopurine or homopyrimidine DNA target sequence, a PNA-DNA-PNA triplex hybrid can form which is extremely stable (Bentin et al., 1996, Biochemistry, 35, 8863-8869, Egholm et al., 1995, Nucleic Acids Res., 23, 217-222, Nielsen et al., Science, 1991, 254, 1497-1500, Demidov et al., Proc.Natl.Acad.Sci., 1995, 92, 2637-2641). They have been shown to be resistant to nuclease and protease digestion (Demidov et al., Biochem.Pharm., 1994, 48, 1010-1013).

In view of protection against cellular nucleases the protection sequences can be any nucleic acid sequences of a length of at least 20 nucleic acids, preferably at least 50 nucleic acids, more preferably at least 100 nucleic acids. These nucleic acids can be digested by nucleases thereby protecting or delaying degradation of the core sequences, i.e promoter sequences, viral sequences and termination sequences of the expression construct.

The linear expression construct can comprise at least one viral gene segment inserted between the poll promoter and the termination signal. For example, a viral cDNA corresponding to each gene in the target virus genome is inserted into a linear expression construct of the invention resulting in a set of linear expression constructs covering the complete viral genome. To amplify these constructs, PCR technology as known in the art can be used, avoiding time-consuming cloning, amplification, sequencing and purification in bacterial host cells.

According to a preferred embodiment the viral segments are derived from viruses of the families of Orthomyxoviridae, Bunyaviridae and Arenaviridae. More preferred, they are derived from influenza, types A, B and C viruses, as well as Thogoto and Dhori viruses and infectious salmon anemia virus.

In case of influenza virus, the viral gene segment can be selected from the group consisting of a PA, PB1, PB2, HA, NA, NP, M or NS gene or part thereof. Alternatively, the viral NS gene segment can be coding for a non-functional NS1 protein. This can be any modification within the NS1 gene, i.e. a substitution, insertion or deletion of nucleic acids. Preferably the modifications of the NS1 gene diminish or eliminate the ability of the NS1 gene product to antagonize the cellular IFN response. Examples for influenza viruses having reduced or no interferon antagonist activity are described in detail in US 6,669,943 or US 6,468,544.

The linear expression construct according to the invention the viral gene segment can also be a cDNA copy or RT-PCR amplification product of said segment

The linear expression constructs of the invention can also be combined into a set of at least two expression constructs. For example, a set of eight linear expression constructs each containing one viral gene segment of PA, PB1, PB2, HA, NA, NP, M and NS or part thereof of influenza virus can be provided.

Alternatively, the linear expression construct can be circularized by peptide linkers and/or overlapping sequences. Various different systems for circularization might be possible like using a 5'oligo with a GGGG extension and a 3'oligo with a CCCC sequence.

Alternatively, after T4 DNA polymerase treatment in presence of dATP and dTTP (to generate sticky ends) could linear constructs be circularized via ligation.

The present invention also provides host cells comprising at least one inventive linear expression construct.

Within the scope of the invention, the term "cells" means the cultivation of individual cells, tissues, organs, insect cells, avian cells, mammalian cells, hybridoma cells, primary cells, continuous cell lines, and/or genetically engineered cells, such as recombinant cells expressing a virus. These can be for example BSC-1 cells, LLC-MK cells, CV-1 cells, CHO cells, COS cells, murine cells, human cells, HeLa cells, 293 cells, VERO cells, MDBK cells, MDCK cells, MDOK cells, CRFK cells, RAF cells, TCMK cells, LLC-PK cells, PK15 cells, WI-38 cells, MRC-5 cells, T-FLY cells, BHK cells, SP2/0 cells, NS0, PerC6 (human retina cells), chicken embryo cells or derivatives, embryonated egg cells, embryonated chicken eggs or derivatives thereof. Preferably the cell line is a VERO cell line.

Besides well known methods to introduce cDNA sequences into expression systems, the present invention also provides a further method for easily constructing the linear expression constructs, wherein the viral segments are provided with complementary sequences overlapping with the poll promoter and poll terminator sequences. In that case, three different fragments are combined, linearized and amplified by PCR.

Using the linear expression construct according to the present invention the transformation and amplification of plasmids in bacterial cells is not required. The linear fragments each containing at least one segment of the complete influenza virus genome or part thereof can be used directly to transfect host cells. The use of these linear constructs provide a system for transfection and expression of virus particles using the construct according to the invention wherein only few days are needed to receive a complete virus particle.

In summary, starting from an influenza virus isolate, transfections can be performed within 2-3 days after receiving the virus.

In contrast, cloning of the plasmids according to the state of the art would require at least 4-5 days without sequencing. Transfection could be performed on day 5. However, several clones for each "new" segment have to be tested to optimise the chance of using a correct plasmid. If sequences are available for the respective virus isolate sequencing of several clones for each segment will further delay the process (provided the sequencing equipment is available) about one day. Thus, transfection could be done on day 6 at the earliest. Complete sequencing without prior sequence information would add 2-3 days for oligonucleotide synthesis

The invention also covers the method for producing a negative strand virus particle comprising culturing a host cell under conditions that permit production of viral proteins and vRNA or cRNA. For example, the linear constructs containing all viral gene segments were used to transfect host cells. Cells were then maintained in culture medium and viral particles were isolated and purified from the culture supernatant.

It covers a method for generating influenza virus particles wherein at least one linear expression construct is directly transfected into animal host cells, and wherein said host cells are cultured under conditions that influenza virus is expressed and virus particles are collected and purified.

Further, a method is also covered, wherein virus particles are incorporated optionally after attenuating or inactivating, into a pharmaceutical composition together with a pharmaceutically acceptable carrier and/or adjuvant as therapeutic or prophylactic medicament. Preferably, the virus particles are used for the production of a medical preparation for therapeutic or prophlyactic treatment of infectious diseases, esp. a .vaccine formulation.

The foregoing description will be more fully understood with reference to the following examples. Such examples are, however, merely representative of methods of practicing one or more embodiments of the present invention and should not be read as limiting the scope of invention.

### Examples

### Example 1: Generation of a linear H3N2 HA expression construct

The HA segment of a Vero cell culture-derived influenza A H3N2 virus was PCR amplified using the oligonucleotides P1 and P2 (F1 in figure 1a). Subsequently, two DNA fragments (F2 and F3 in figure 1) derived from pHW2000 (Hoffmann et al. 2000, Proc Natl Acad Sci U S A. 97:6108-13) were fused to the HA PCR product by means of overlapping PCR (see figure 1 b). The first DNA fragment (F2) comprises the CMV promoter and the Poll terminator, the second one (F3) comprises the human Poll promoter and the BGH polyA signal. To facilitate generation of the overlapping PCR products, oligonucleotides used for HA amplification were extended on their 5' ends in that P1 contains a sequence complementary to the Poll terminator and P2 contains a sequence complementary to the Poll promoter (see figure 1 a). Similarly, the primers P3 and P5 used for generation of the fragments F1 and F2 were extended on their 5' termini to contain sequences complementary to the 5' and 3' end of the HA (see figure 1 a). Fragments F2 and F3 contain protection sequences derived from sequence described in ? the pHW2000 backbone. These sequences are not directly involved in transcription of mRNA and vRNA but reduce degradation of the bidirectional expression cassette by exonucleases.

Viral RNA was extracted from a Vero cell culture-derived influenza A H3N2 virus using a Qiagen ViralAmp kit and reverse transcribed using the Uni12 oligonucleotide as described previously (Hoffmann et al. 2001, Arch Virol. 146:2275-89).

The HA segment was amplified with the oligonucleotides shown in the table 1 using a mixture of Pfu Turbo DNA polymerase and Taq DNA polymerase:

**Table 1:**

| | |
|---|---|
| P1 | 5'-CGAAGTTGGGGGGG***AGCAAAAGCAGGGGATAATTCTATTAAC***-3' (SEQ ID No. 1) |
| P2 | 5'-GCCGCCGGGTT***ATTAGTAGAAACAAGGGTGTTTTTAATTAATGC***-3' (SEQ ID No. 2) |

Nucleotides corresponding to the H3 sequence are shown in italic bold letters, nucleotides homologous to the Poll terminator (P1) and the Poll promoter (P2) are shown in standard capital letters.

The HA F4 PCR product was purified using a Qiaquick PCR Purification kit (Qiagen). PCR fragments F2 and F3 were amplified from pHW2000 plasmid DNA with the primer pairs P3+P4 and P5+P6 (see table 2 and figure 1a), respectively using a mixture of Pfu Turbo DNA polymerase and Taq DNA polymerase. PCR products F2 and F3 were purified using a QIAquick PCR Purification kit (Qiagen)

**Table 2:**

| | |
|---|---|
| P3 | 5'-***CCTGCTTTTGCT***CCCCCCCAACTTCGGAGGTC-3' (SEQ ID No. 3) |
| P4 | 5'-GGGGTATCAGGGTTATTGTCTCATGAGCGGATAC-3' (SEQ ID No. 4) |
| P5 | 5'-***CCTTGTTTCTACT***AATAACCCGGCGGCCCAAAATGC-3' (SEQ ID No. 5) |
| P6 | 5'-CCCCTTGGCCGATTCATTAATGCAGCTGGTTC3' (SEQ ID No. 6) |

For P3 and P5 nucleotides corresponding to the H3 sequence are shown in italic bold letters, nucleotides complementary to pHW2000 are shown in standard capital letters. For P4 and P6 all nucleotides except the four nucleotides at the 5' ends correspond to pHW2000.

For generation of the full length PCR product (F4) containing the HA, the CMV promoter, the Poll terminator, the Poll promoter and the BGH polyA signal, fragments F1, F2 and F3 were combined and amplified by overlapping PCR with the primers P4 and P6 using a mixture of Pfu Turbo DNA polymerase and Taq DNA polymerase.

Figure 1 shows a schematic diagram of the generation of linear bidirectional expression constructs.

Figure 1a) schematically discloses Fragments F1, F2 and F3 generated separately by PCR amplification.

Fragment F1 contains the respective viral segment and contains extensions complementary to the Poll promoter and Poll terminator. Fragment F2 contains the CMV promoter and the Poll terminator as well as an extension complementary to the respective viral segment. Fragment F3 contains the Poll promoter and the BGH poly adenylation signal as well as an extension complementary to the respective viral segment. Oligonucleotides P1 and P2 used for PCR amplification of F1 fragments are complementary to the respective viral segment. P1 contains a 5' extension complementary to the Poll terminator, P2 contains a 5'extension complementary to the Poll promoter.

Oligonucleotides P3 and P4 are used for PCR amplification of F2 fragments with P3 containing a 5'extension complementary to the respective viral segment. Oligonucleotides P5 and P6 are used for PCR amplification of F3 fragment with P5 containing a 5'extension complementary to the respective viral segment.

Protection sequences are derived from the pHW2000 backbone and do not contain sequences directly involved in mRNA or vRNA transcription.

### Example 2: Influenza A virus rescue using a linear HA expression construct

Six influenza A H3N2 virus isolates were grown on MDCK cells. The HA segments were PCR amplified similar (F1 in figure 1) and purified via agarose gel electrophoresis using a Qiaex II kit (Qiagen).

Fragments F2 and F3 were fused to F1 as described in example 1 to yield the full length expression constructs F4. Following purification via agarose gel electrophoresis fragments F4 were PCR reamplified to yield sufficient amounts of DNA for transfection. Finally, the F4 HA DNA fragments were used together with a set of seven plasmids (pHW2000 derivatives) that contain the remaining segments of a Vero adapted Influenza A H1N1 deINS1 strain (GHB01) for virus rescue on Vero cells.

Figure 1b discloses fragment F4 generated by overlapping PCR using the oligonucleotides P4 and P6.

Generation of F4 HA DNA fragments was done similarly to the procedure described in example 1. A total amount of 10-20µg F4 HA DNA for each viral isolate were first purified using a Qiaquick kit (Qiagen) and subsequently via a Qiagen Endofree Plasmid kit.

Vero cells were maintained in DMEM/F12 medium containing 10% foetal calf serum and 1% Glutamax-I supplement at 37°C.

For virus generation seven derivatives from the published sequence pHW2000 (Hoffmann et al., see above) were generated containing the segments PA, PB1, PB2, NA, M, NP and deINS1 derived from GHB01 as well as a protein expression plasmid coding for Influenza A PR8 NS1 (pCAGGS-NS1(SAM); (Salvatore et al. 2002, J Virol. 76:1206-12)) were used together with the respective F4 HA DNA fragment for cotransfection of Vero cells. Following transfection, to support virus replication, Vero cells were cultured in serum-free medium (Opti-Pro; Invitrogen) in the presence of 5µg/ml trypsin. Three to four days after transfection 50-100% CPE was observed and rescued viruses were frozen or further amplified on Vero cells.

Thus virus rescue for influenza A with one bidirectional expression plasmid replaced by a linear PCR product is feasible.

### Example 3: Influenza A virus rescue entirely from linear expression constructs

Eight linear expression constructs (F4) for a Vero cell-adapted Influenza A H1 N1 deINS1 virus (GHB01) were generated by PCR amplification. Eight pHW2000 derivatives that contain the segments of GHB01 served as templates for PCR.

Sufficient amounts of F4 fragments were generated for each segment and subsequently used for virus rescue on Vero cells.

F4 DNA fragment generation was done for each of the eight segments by direct PCR amplification of each whole bidirectional expression cassette containing the respective influenza segment using the respective pHW2000 derivative as template. PCR amplification was performed with oligonucleotides P4 and P6 (shown in the table 3) using a mixture of Pfu DNA Turbo polymerase and Taq DNA polymerase.

**Table 3:**

| | |
|---|---|
| P4 | 5'-GGGGTATCAGGGTTATTGTCTCATGAGCGGATAC-3' (SEQ ID No. 4) |
| P6 | 5'-CCCCTTGGCCGATTCATTAATGCAGCTGGTTC3' (SEQ ID No. 6) |

Sufficient amounts of F4 PCR product (10-20µ) were generated for each segment and purified first using a Qiaquick kit (Qiagen) and subsequently via a Qiagen Endofree Plasmid kit.

Vero cells were transfected with equal amounts of the eight F4 DNA fragments and the NS1 expression plasmid pCAGGS-NS1(SAM). Following transfection, to support virus replication, Vero cells were cultured in serum-free medium (Opti-Pro; Invitrogen) in the presence of 5µg/ml trypsin.

Complete CPE was observed four days after transfection. Thus, virus rescue from only linear bidirectional influenza A expression constructs is feasible.

## Claims

1. A linear expression construct free of any amplification and/or selection sequences comprising an RNA polymerase I (poll) promoter and a poll termination signal, inserted between a RNA polymerase II (poIII) promoter and a polyadenylation signal.

2. The linear expression construct according to claim 1 comprising additional protection sequences at the N- and/or C-terminus of the construct.

3. The linear expression construct according to any one of claims 1 or 2, wherein the protection sequences can be any sequences not directly involved in transcription of the vRNA.

4. The linear expression construct according to any one of claims 1 to 3 comprising at least one viral gene segment inserted between the poll promoter and the poll termination signal.

5. The linear expression construct according to claim 4 wherein the viral segment is from influenza virus of type A, B or C.

6. The linear expression construct according to any one of claims 4 or 5 wherein the viral gene segment is selected from the group consisting of an PA, PB1, PB2, HA, NA, NP, M, NS gene segment or part thereof of influenza virus.

7. The linear expression construct according to any one of claims 4 to 6 wherein the viral NS gene segment is expressing a non-functional NS1 protein

8. The linear expression construct according to any one of claims 4 to 7 wherein the viral gene segment is a cDNA copy or RT-PCR amplification product of said segment.

9. A set of linear expression constructs containing at least two expression constructs according to claims 1 to 8.

10. A set of eight linear expression constructs according to claim 9 each containing at least one viral gene segment of PA, PB1, PB2, HA, NA, NP, M and NS or part thereof of influenza virus.

11. Host cell comprising at least one linear expression construct according to any one of claims 1 to 10.

12. A method for producing a negative strand RNA virion comprising culturing the host cell of claim 11 under conditions that permit production of viral proteins and vRNA or cRNA.

13. A method for generating influenza virus particles wherein at least one linear expression construct is directly transfected into animal host cells, and wherein said host cells are cultured under conditions that influenza virus is expressed and virus particles are collected and purified.

14. A method according to any one of claims 12 or 13 wherein said virus particles are incorporated optionally after attenuating or inactivating, into a pharmaceutical composition together with a pharmaceutically acceptable carrier.

15. Use of virus particle produced by using an expression construct according to any one of claims 1 to 8 for the production of a medical preparation for therapeutic or prophlyactic treatment of infectious diseases.
